# EUROPÄISCHE PATENTSCHRIFT

(11) **EP 0 773 246 B1**
(45) Veröffentlichungstag und Bekanntmachung des Hinweises auf die Patenterteilung: **29.09.1999**
(21) Anmeldenummer: 96117641.9
(22) Anmeldetag: 04.11.1996
(51) Int. Cl.: C08G 18/12, C08G 18/66, A61K 7/06

(54) **Wasserlösliche oder wasserdispergierbare Pfropfpolymere, ihre Herstellung und ihre Verwendung**
Water-soluble or water-dispersible graft polymers, their preparation and use
Polymères greffés solubles ou dispersables dans l'eau, leur préparation et utilisation

(30) Priorität: 08.11.1995 DE 19541658
(43) Veröffentlichungstag der Anmeldung: 14.05.1997
(73) Patentinhaber: BASF AKTIENGESELLSCHAFT, 67056 Ludwigshafen (DE)
(72) Erfinder: Nguyen Kim, Son, Dr., 69502 Hemsbach (DE); Sanner, Axel, Dr., 67227 Frankenthal (DE); Hössel, Peter, Dr., 67105 Schifferstadt (DE)

(56) Entgegenhaltungen:
- EP-A- 0 619 111
- DE-A- 3 831 169
- FR-A- 2 152 240
- GB-A- 2 086 395

## Beschreibung

Die vorliegende Erfindung betrifft wasserlösliche oder wasserdispergierbare Pfropfpolymere, ihre Herstellung und ihre Verwendung in der Kosmetik.

In Wasser lösliche oder in Wasser dispergierbare Polymere, beispielsweise Polyester, Polyamide oder Polyurethane, gewinnen aufgrund ihrer niedrigen Viskosität immer mehr an Bedeutung. So sind wasserlösliche Polyurethane, die Carboxylgruppen aufweisende Diole einpolymerisiert enthalten, aus der US-A 3,412,054 und 3,658,939 bekannt. Sie werden als Klebstoff, Beschichtungsmittel und in Drucktinten verwendet. Sulfonat- und/oder Carboxylatgruppen enthaltende Polyurethane, die in Wasser dispergierbar sind, sind aus der DE-A 15 70 615 bekannt. Sie werden beispielsweise zur Beschichtung und zum Imprägnieren von Textilien, Leder, Papier, Holz und Metallen verwendet. Aus den Schutzrechten US-A 4,300,580, US-A 3,734,874, DE-A 26 33 418 und WO-A 89/07118 sind NaSO₃-Gruppen enthaltende Polyester bekannt, deren Hauptkette durch Kondensationsreaktion aufgebaut ist und die durch Hydrolyse der Estergruppierungen zu kürzeren Segmenten abgebaut werden können.

Weiter ist bekannt, daß Maleinsäureanhydrid und Trimellitsäureanhydrid zur Herstellung von wasserlöslichen Estern verwendet werden können. Die Anhydridgruppierung stellt Carboxylgruppen zur Verfügung, welche durch Neutralisation mit Aminen, Metallhydroxiden und Metallcarbonaten in Carboxylatgruppen überführt werden, wodurch Wasserlöslichkeit erzielt wird. Aus der DE-A 26 37 167 und der US-A 3,523,998 ist bekannt, daß auch Polycarbonsäuren und ihre Anhydride als Polymerisatkomponenten dazu beitragen können, Polyester wasserlöslich zu machen. Die DE-A 21 44 878 beschreibt Polyurethane, bei denen es sich um Umsetzungsprodukte aus aufgeschlossenem Kasein, in Wasser dispergierbaren Polyurethanen und Formaldehyd handelt. Als Polyurethankomponente wird unter anderem ein Latex eingesetzt, der erhältlich ist durch Umsetzung eines Polyurethanprepolymers mit einer Natriumtaurinlösung. Der Latex besitzt ein relativ niedriges Molekulargewicht und einen geringen Gehalt an ionogenen bzw. ionischen Gruppen, weil er neben den Sulfonatgruppen aus dem Taurin keine weiteren ionogenen bzw. ionischen Gruppen enthält. Ein aus dem Latex erhaltener Film ist daher in Wasser ohne Dispergiermittel nicht löslich. Der erhaltene Latex wird dann mit Kasein und Formaldehyd zu dem erwähnten Umsetzungsprodukt zur Reaktion gebracht. In DE 22 61 056 werden Polymere beschrieben, die durch Reaktion eines Polymers mit alkoholischer Funktion mit einem Protein und einem Isocyanat entstehen.

Diese hochvernetzten Polymere sind nicht wasserlöslich oder in Wasser dispergierbar und eignen sich als Lederbeschichtungsmittel und für die Herstellung von Schuhsohlen.

Eine kosmetische Anwendung derartiger Polymere ist bisher jedoch noch nicht beschrieben.

In der Kosmetik werden Filmbildnerpolymere zur Festigung, Strukturverbesserung und Formgebung der Haare verwendet. Die Haarbehandlungsmittel enthalten im allgemeinen eine Lösung des Filmbildners in einem Alkohol oder einem Gemisch aus Alkohol und Wasser.

Die US-A 4,743,673 beschreibt hydrophile Polyurethanpolymere mit Carboxygruppen im Polymerrückgrat. Diese Polyurethane sind aufgebaut aus einer Polyolkomponente, bei der es sich um ein Alkylenglykol, ein Polyoxyalkylenglykol, oder ein lineares Polyesterdiol handeln kann, einer Carbonsäureesterkomponente mit Hydroxy- oder Aminogruppen und einem organischen Isocyanat oder Isocyanat-Vorläufer. Das Polyurethan enthält also an das Polymerrückgrat gebundene Estergruppen, die anschließend durch 30- bis 60-minütiges Erhitzen mit einer starken Base, wie Natrium- oder Kaliumhydroxid, unter Rückfluß verseift werden, Nach Überführung der Carboxylatgruppen in die Säureform und erneuter Neutralisation mit Ammoniak erhält man aus der Lösung einen Film. Dieser ist nicht mehr in Wasser, sondern nur noch in niedrigen aliphatischen Alkoholen und anderen Lösungsmitteln löslich. Insbesondere bei Verwendung eines Polyesterdiols als Polyolkomponente erfolgt aufgrund der Behandlung mit der starken Base unter Rückflußbedingungen nicht nur eine Verseifung der Estergruppen der Carbonsäureesterkomponente, sondern auch eine Verseifung der in der Polyurethankette enthaltenen Estergruppierungen. Es kommt somit zur Spaltung der Polyurethankette und zu einer drastischen Verringerung des Molekulargewichts der Polyurethane. Eine Anwendung der Polyurethane in Haarsprays ist zwar erwähnt. In der Praxis sind die mit diesen Polyurethanen erhaltenen Filme für die Haarkosmetik aber nicht brauchbar, weil sie entweder wasserunlöslich sind oder ein zu geringes Molekulargewicht und somit unzureichende Festigungswirkung besitzen.

Die DE-A 42 25 045 beschreibt die Verwendung von wasserlöslichen oder in Wasser dispergierbaren, anionischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Sie besitzen eine Glastemperatur von mindestens 15°C und Säurezahlen von 12 bis 150. Als Komponente a) kommen vorzugsweise Polyethylenglykol, Neopentylglykol und Polyesterole zur Anwendung. Bevorzugte Komponenten b) sind Dimethylolpropansäure, ein Kondensat aus Pyromellithsäuredianhydrid und Neopentylglykol und ein Kondensat aus 5-Natriumsulfonatoisophthalsäure mit Neopentylglykol.

Die DE-A 42 41 118 beschreibt die Verwendung von kationischen Polyurethanen und Polyharnstoffen als Hilfsmittel in kosmetischen und pharmazeutischen Zubereitungen. Sie finden insbesondere Anwendung als Filmbildner in Haarfestigern. Sie sind aufgebaut aus
a) mindestens einem Diisocyanat, welches bereits vorher mit einer oder mehreren Verbindungen, die zwei oder mehrere aktive Wasserstoffatome pro Molekül enthalten, umgesetzt sein kann und
b) mindestens einem Diol, primären oder sekundären Aminoalkohol, primären oder sekundären Diamin oder primären oder sekundären Triamin mit einem oder mehreren tertiären, quartären oder protonierten tertiären Aminstickstoffatomen.

Die Polymere besitzen eine Glasübergangstemperatur von mindestens 25°C und eine Aminzahl von 50 bis 200, bezogen auf die nicht-quaternisierten oder protonierten Verbindungen.

Die EP-A 619 111 beschreibt die Verwendung von Polyurethanen mit Carboxylatgruppen in Haarfixierungsmitteln. Als Verbindung, welche die Carboxylatgruppen zur Verfügung stellt, enthalten diese Polyurethane eine Verbindung der Formel 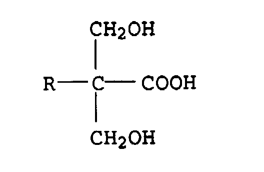 worin R für ein Wasserstoffatom oder eine C₁-C₂₀-Alkylgruppe steht. Zumindest ein Teil der Carbonsäuregruppen wird dabei mit einer organischen oder anorganischen Base neutralisiert, um die Anzahl an Carboxylatgruppen zur Verfügung zu stellen, die erforderlich ist, um das Polyurethan in Wasser oder einem Gemisch aus Wasser und einem polaren organischen Lösungsmittel löslich zu machen.

Haarfestigungsmittel werden im allgemeinen in Form von wäßrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, daß sie aus dem Haar ausgewaschen werden können, andererseits aber sollen sie hydrophob sein, damit die mit den polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht (K-Wert > 25) und eine relativ hohe Glastemperatur (mindestens 15°C) besitzen. Polymere, welche diese Forderungen erfüllen, sind aber wenig hydrophil. Das hat zur Folge, daß sie eine schlechte Auswaschbarkeit besitzen und außerdem nur in Form alkoholreicher Formulierungen zur Anwendung kommen können.

Bei der Formulierung von Haarfestigern ist außerdem zu berücksichtigen, daß aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Die in den oben erwähnten Publikationen beschriebenen Polymere erfüllen diese einander entgegenstehenden Anforderungen nur teilweise. So besitzen die in der DE-A 42 25 045 und 42 41 118 sowie in der EP-A 619 111 beschriebenen Polymere aufgrund ihres hohen Molekulargewichtes einerseits die gewünschte Festigerwirkung. Andererseits aber sind sie nur unzureichend auswaschbar und nur in Formulierungen anwendbar, deren VOC-Gehalt größer als 80 % ist. Die in der US 4,743,673 beschriebenen Polymere wiederum sind entweder wasserunlöslich und damit nicht auswaschbar oder sie besitzen aufgrund ihres geringen Molekulargewichtes nicht die erforderliche Festigerwirkung.

Der vorliegenden Erfindung liegt daher die Aufgabe zugrunde, Haarbehandlungsmittel zur Verfügung zu stellen, die einerseits als Haarfestiger brauchbar sind, andererseits aber auch verbesserte Auswaschbarkeit (Redispergierbarkeit) sowie eine gute Bioabbaubarkeit besitzen. Außerdem sollen diese Haarbehandlungsmittel einen niedrigen VOC-Gehalt (< 60 %) aufweisen.

Überraschenderweise wurde nun gefunden, daß diese Aufgabe durch wasserlösliche bzw. durch Wasser dispergierbare Pfropfpolymere gelöst wird, welche das Umsetzungsprodukt aus einem Urethanpräpolymer mit endständigen Isocyanatgruppen mit einem freie Aminogruppen enthaltenden Protein darstellen.

Gegenstand der vorliegenden Erfindung sind daher wasserlösliche oder wasserdispergierbare Pfropfpolymere aus
A) eine in Wasser löslichen oder dispergierbaren Urethanpräpolymer mit endständigen Isocyanatgruppen und
B) einem freie Aminogruppen enthaltenden Protein,
sowie die Salze davon.

Das Protein B) reagiert über seine freien Aminogruppen mit den endständigen Isocyanatgruppen des Urethanpräpolymers, so daß das Protein über eine Harnstoffgruppierung an das Polyurethan gebunden ist. Die erfindungsgemäßen Polyurethane weisen also endständige, von dem Protein abgeleitete Reste auf.

Um eine gute Wasserdispergierbarkeit zu erreichen, wählt man das Verhältnis zwischen A) und B) so, daß der Gehalt an NCO-Äquivalenten in A) kleiner ist als der Gehalt an NH₂-Äquivalenten in B).

Als Protein B) sind ganz allgemein alle Proteine, die über freie Aminoseitengruppen verfügen, geeignet. Es können sowohl reine Proteine oder auch Gemische verschiedener Proteine verwendet werden. Weiterhin sind auch Abbauprodukte von Proteinen, beispielsweise partiell hydrolysierte Proteine einsetzbar. Die Abbauprodukte können durch enzymatischen Abbau, z.B. mittels Protease, oder durch chemischen Abbau, z.B. Basen- oder Säurespaltung erhalten werden.

Bevorzugt werden als Proteine B) Caseine und deren Hydrolyseprodukte eingesetzt.

Die Proteine B) werden in der Regel als wäßrige oder wäßrig-alkoholische Lösung gegebenenfalls nach Zugabe einer Base mit dem Polyurethanpräpolymer A) zur Reaktion gebracht.

Die Umsetzung der Proteinlösung mit dem Polyurethanpräpolymer A) wird in der Regel bei einem pH-Wert > 7 durchgeführt.

Besonders bevorzugt setzt man Proteinlösungen zur Reaktion mit A) ein, die noch zusätzlich ein tertiäres Amin als Base, insbesondere Triethanolamin, Ethyldiethanolamin oder Diethylethanolamin, enthalten. Das tertiäre Amin dient zur Neutralisation der Carboxylfunktionen des Proteins.

Erfindungsgemäß brauchbare Polyurethan-Präpolymere A) sind bekannt. Es handelt sich dabei um Polyurethane, welche ionogene bzw. ionische, an die Polymerkette gebundene Gruppen aufweisen, damit die Polyurethane in Wasser löslich bzw. dispergierbar sind. Bei diesen Gruppen handelt es sich vorzugsweise um Carboxylatgruppen und/oder Sulfonatgruppen und/oder stickstoffhaltige Gruppen. Derartige Polymere sind beispielsweise beschrieben in der US-A 3,475,206 und 3,412,054 sowie in der DE-A 15 80 615. Vorzugsweise verwendet man jedoch die in der DE-A 42 25 045, DE-A 42 41 118 und EP-A 619 111 beschriebenen Polyurethane. Es handelt sich dabei um die folgenden:
1. In Wasser lösliche oder dispergierbare anionische Polyurethane aus
   a) mindestens einer Verbindung, die zwei oder mehrere aktive Wasserstoffatome pro Molekül enthält,
   b) mindestens einer Verbindung, die zwei oder mehrere aktive Wasserstoffatome und mindestens eine Säure-, eine tertiäre Amin- oder eine ionogene bzw. ionische Gruppe pro Molekül aufweist, und
   c) mindestens einem Diisocyanat,
      und die Salze davon,
   wobei das Verhältnis von NCO-Äquivalent zu Äquivalent aktivem Wasserstoff 1:1 bis 1,2:1 beträgt (d.h. Verhältnis c) : (a) + (b)).
   Bei der Komponente a) handelt es sich insbesondere um Diole, Diamine, Polyesterdiole, Polyetherdiole oder deren Mischungen mit einem zahlenmittleren Molekulargewicht von jeweils bis zu 3000, wobei bis zu 3 Mol.-% der genannten Verbindungen durch Triole oder Triamine ersetzt sein können.
   Brauchbare Diole sind z.B. Ethylenglykol, Propylenglykol, Butylenglykol, Neopentylglykol, Polyetherole, wie Polyethylenglykole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglykol, Di-, Tri-, Tetra-, Penta oder Hexaethylenglykol. Brauchbare Diole sind außerdem Poly(α-hydroxycarbonsäure)diole der Formeln 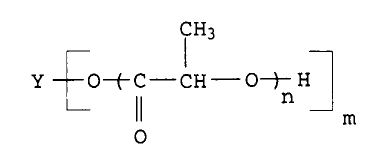 worin Y für den Rest eines 2- bis 4-wertigen Alkohols steht, n für 1 bis 50 steht und m für 1 bis 4 steht, und der Formel 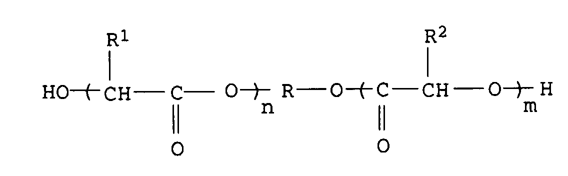 worin R¹ und R² für H, C₁-C₅-Alkyl oder Aryl (vorzugsweise Phenyl) stehen, R für einen Rest eines Diols (C₂-C₈-Alkylenrest) steht und n und m für 1 bis 30 stehen.
   Als Komponente a) brauchbar sind auch Silikonverbindungen der Formel 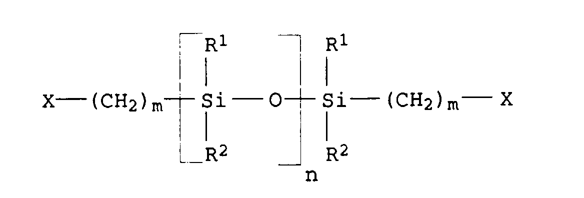 worin R¹ und R², die gleich oder verschieden sein können, für C₁-C₄-Alkyl, Benzyl oder Phenyl, vorzugsweise Methyl-, stehen,
   die Reste X, die gleich oder verschieden sein können, für OH oder NH2 stehen,
   m für 2 bis 10 steht, und
   n für 3 bis 50 steht.
   Diese Silikonverbindungen können in einer Menge von bis zu 50 Gew.-%, bezogen auf das Gesamtgewicht der Komponenten a) und b) zur Anwendung kommen.
   Bei der Komponente b) handelt es sich insbesondere um Dimethylolpropansäure oder Verbindungen der Formeln 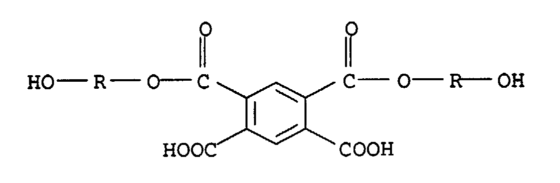 und/oder 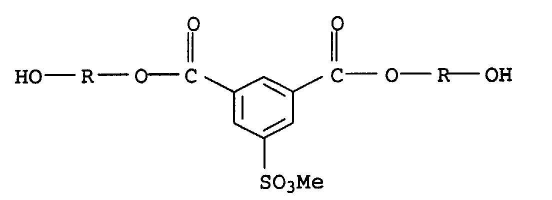 worin R jeweils für eine C₂-C₁₈-Alkylengruppen steht und Me für Na oder K steht.
   Bei der Komponente c) handelt es sich insbesondere um Hexamethylendiisocyanat, Isophorondiisocyanat und/oder Toluylendiisocyanat.
   Die Polyurethanpräpolymere A) sind dadurch erhältlich, daß man die Verbindungen der Gruppen a) und b) unter einer Inertgasatmosphäre in einem inerten Lösemittel bei Temperaturen von 70 bis 130°C mit den Verbindungen der Gruppe c) umsetzt. Diese Umsetzung kann gegebenenfalls in Gegenwart von Kettenverlängern durchgeführt werden, um Polyurethane mit höheren Molekulargewichten herzustellen. Dabei werden die Komponenten in solchen Mengen eingesetzt, daß das Verhältnis von NCO-Äquivalent zu OH-Äquivalent größer als 1 ist und bis zu 1,2 betragen kann. Vorzugsweise liegt das Verhältnis im Bereich von 1,02 bis 1,12. Die Säurezahl der Polyurethane wird von der Zusammensetzung und der Konzentration der Verbindungen der Komponente b) in der Mischung aus den Komponenten (a)+(b) bestimmt. Die Polyurethane haben K-Werte nach H. Fikentscher (bestimmt in 0,1 gew.-%igen Lösungen in N-Methylpyrrolidon bei 25°C und pH 7) von 15 bis 100, vorzugsweise 20 bis 50.
   Diese Polymere und ihre Herstellung sind in der DE-A-42 25 045 näher beschrieben, auf die hiermit Bezug genommen wird.
2. In Wasser lösliche oder dispergierbare, kationische Polyurethane und Polyharnstoffe aus
   a) mindestens einem Diisocyanat, welches bereits vorher mit einer oder mehreren Verbindungen, die zwei oder mehrere aktive Wasserstoffatome pro Molekül enthalten, umgesetzt worden sein kann, und
   b) mindestens einem Diol, primären oder sekundären Aminoalkohol, primären oder sekundären Diamin oder primären oder sekundären Triamin mit einem oder mehreren tertiären, quarternären oder protonierten tertiären Aminstickstoffatomen,
      die eine Aminzahl von 50 bis 200, bezogen auf die nichtquaternisierten oder protonierten Verbindungen, aufweisen, und die Salze davon.

   Bevorzugte Diisocyanate sind wie oben unter 1. angegeben. Verbindungen mit zwei oder mehreren aktiven Wasserstoffatomen sind Diole, Aminoalkohole, Diamine, Polyesterole, Polyamiddiamine und Polyetherole. Geeignete Diole sind wie oben unter 1. angegeben.
   Geeignete Aminoalkohole sind z.B. 2-Aminoethanol, 2-(N-Methylamino)ethanol, 3-Aminopropanol oder 4-Aminobutanol.
   Geeignete Diamine sind z.B. Ethylendiamin, Propylendiamin, 1,4-Diaminobutan und 1,6-Diaminohexan sowie α,ω-Diamine, die durch Aminierung von Polyalkylenoxiden mit Ammoniak herstellbar sind.
   Polyesterole sind z.B. Umsetzungsprodukte aus Phthalsäure und Diethylenglykol, Isophthalsäure und 1,4-Butandiol, Isophthalsäure/Adipinsäure und 1,6-Hexandiol sowie Adipinsäure und Ethylenglykol.
   Brauchbare Verbindungen mit aktiven H-Atomen sind auch mindestens 5 mol-% eines Poly(milchsäureesterdiols) der allgemeinen Formel 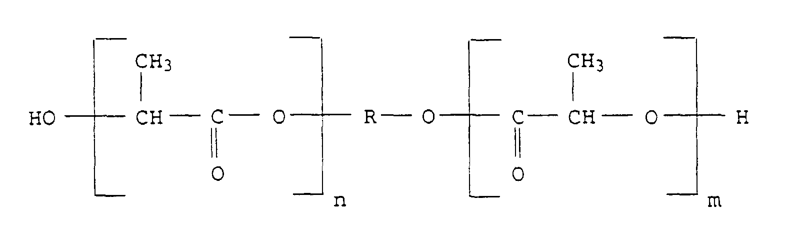 eines Poly(ε-caprolactondiols) der allgemeinen Formel II 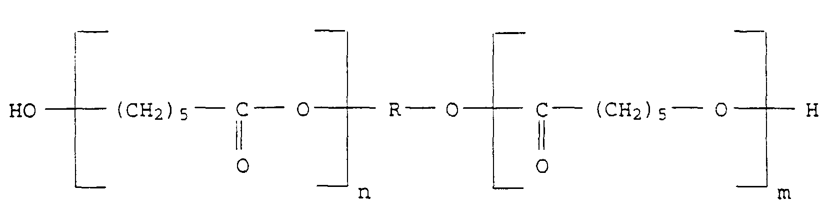 oder eines Polyamiddiamins der allgemeinen Formel III 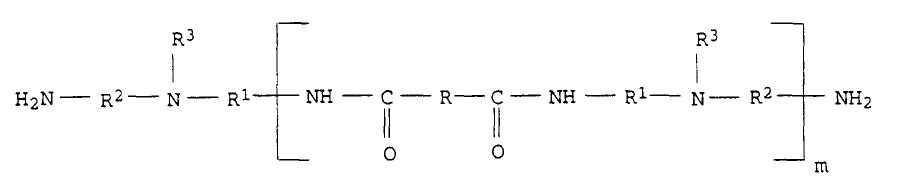 in denen
   - R: C₂- bis C₈-Alkylen, C₅- bis C₈-Cycloalkylen oder Phenylen bezeichnet,
   - R¹ und R²: C₂- bis C₈-Alkylen bedeuten,
   - R³: für C₁- bis C₄-Alkyl, Phenyl oder C₇- bis C₁₀-Phenylalkyl steht und
   - n und m: jeweils eine Zahl von 1 bis 30 bezeichnet.

   Bevorzugte Verbindungen (b) sind Verbindungen der allgemeinen Formeln IV bis XI 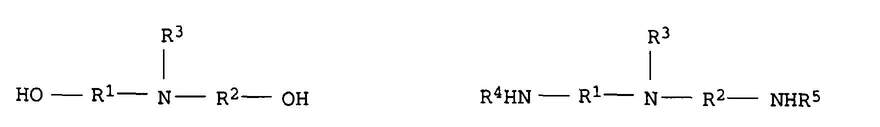 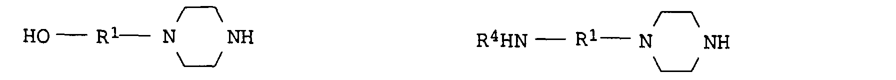 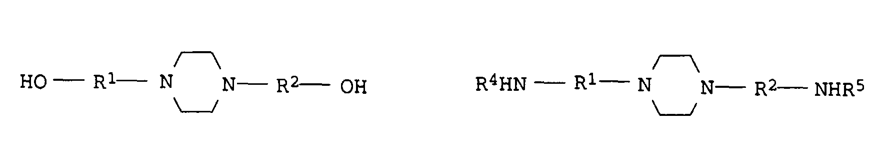 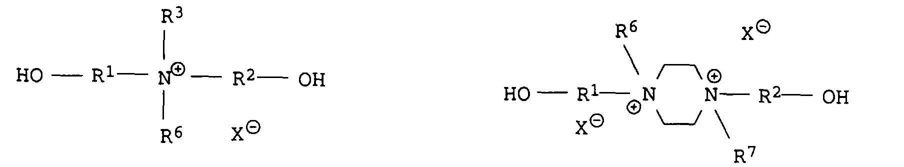 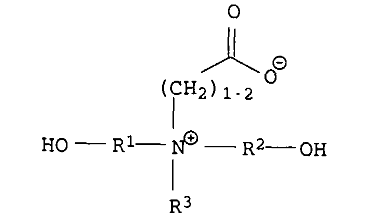 in denen
   R¹ und R² C₂- bis C₈-Alkylen bedeuten,
   R³, R⁶ und R⁷ für C₁- bis C₄-Alkyl, Phenyl oder C₇- bis C₁₀-Phenylalkyl stehen,
   R⁴ und R⁵ Wasserstoff oder C₁- bis C₄-Alkyl bezeichnen und
   x⊖ für Chlorid, Bromid, Iodid, C₁- bis C₄-Alkylsulfat oder die halbe stöchiometrische Menge Sulfat steht.

   Die Herstellung der Polyurethane erfolgt wie oben unter 1. beschrieben.
   Diese Polymere und ihre Herstellung sind in der DE-A-42 41 118 näher beschrieben, auf die hiermit Bezug genommen wird.
3. Lineare Polyurethane mit Carboxylatgruppen aus
   a) einer 2,2-Hydroxymethyl-substituierten Carbonsäure der Formel 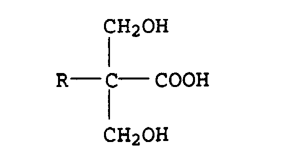 worin R für ein Wasserstoffatom oder eine C₁- bis C₂₀-Alkylgruppe steht, die in einer Menge verwendet wird, welche ausreicht, daß in dem Polyurethan 0,35 bis 2,25 milliäquivalente Carboxylgruppen pro g Polyurethan vorhanden sind,
   b) 10 bis 90 Gew.-%, bezogen auf das Gewicht des Polyurethans, einer oder mehrerer organischer Verbindungen mit nicht mehr als zwei aktiven Wasserstoffatomen und
   c) einem oder mehreren organischen Diisocyanaten.
   Die im Polyurethan enthaltenen Carboxylgruppen werden abschließend mit einer geeigneten Base zumindest teilweise neutralisiert. Diese Polymere und ihre Herstellung sind in der EP-A-619 111 näher beschrieben, auf die hiermit in vollem Umfang Bezug genommen wird.

Es ist klar, daß für die Herstellung des Polyurethanpräpolymers das Diisocyanat im Überschuß verwendet werden muß, um ein Polyurethanpräpolymer mit endständigen Isocyanatgruppen zu erhalten. Die erfindungsgemäßen Polymere besitzen vorzugsweise einen K-Wert von 15 bis 100, insbesondere 20 bis 50.

Die Herstellung der erfindungsgemäßen Pfropfpolymere erfolgt durch Umsetzung des Polyurethanpräpolymers A) mit dem Protein B). Die Umsetzung erfolgt in einer Weise, wie sie aus dem Stand der Technik für das Abstoppen der Polyurethanpolymerisation mit Aminen bekannt ist. Noch vorhandene Isocyanatgruppen werden abschließend durch Zusatz von Aminen, z.B. 2-Amino-2-methyl-1-propanol, inaktiviert.

Vorzugsweise setzt man das Protein in Form einer wäßrigen oder wäßrig-alkoholischen Lösung mit einem pH > 7,0 ein, um die Reaktionsfähigkeit des Proteins zu erhöhen. Die Einstellung des pH's kann in üblicher Weise erfolgen, beispielsweise mit einem Alkalihydroxid, wie NaOH oder KOH oder vorzugsweise mit einem tertiären Amin, wie Triethylamin, einem C₁- bis C₆-Alkyldiethanolamin, z.B. Methyl- oder Ethyldiethanolamin oder einem Di-C₁-C₆-Alkylethanolamin.

Die erfindungsgemäßen Pfropfpolymere sind als Hilfsmittel für die Textil-, Papier- und Lederherstellung sowie in der Kosmetik und Pharmazie brauchbar. Sie sind insbesondere in der Haarkosmetik brauchbar und finden Anwendung als Haarfestiger. Daneben können sie auch in Cremes und als Tablettenüberzugsmittel und Tablettenbindemittel verwendet werden.

Gegenstand der vorliegenden Erfindung ist auch ein Haarbehandlungsmittel, das die erfindungsgemäßen Polyurethane enthält. Im allgemeinen enthält das Mittel die Polyurethane in einer Menge im Bereich von 0,2 bis 20 Gew.-%, bezogen auf das Gesamtgewicht des Mittels.

Die erfindungsgemäßen Haarbehandlungsmittel liegen üblicherweise in Form einer wäßrigen Dispersion oder in Form einer wäßrig-alkoholischen Lösung vor. Beispiele geeigneter Alkohole sind Ethanol, Propanol, Isopropanol, n-Butanol, Ethylenglykol, Propylenglykol, 1,3-Butylenglykol oder ein Polyglykol (z.B. mit einem Molekulargewicht von 200).

Weiter enthalten die erfindungsgemäßen Haarbehandlungsmittel im allgemeinen übliche kosmetische Hilfsstoffe, beispielsweise Weichmacher, wie Glycerin und Glykol; Silikone; Emollienzien; Parfums; UV-Absorber; Farbstoffe; Verdickungsmittel; antistatische Mittel; Mittel zur Verbesserung der Kämmbarkeit; Konservierungsmittel und Schaumstabilisatoren.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel wird so gering wie möglich gehalten, um den VOC-Gehalt nicht unnötig zu erhöhen. Sie beträgt im allgemeinen nicht mehr als 40 Gew.-%, bezogen auf das Gesamtgewicht des Mittels.

Neben den erfindungsgemäßen Pfropfpolymeren können die Haarbehandlungsmittel auch noch weitere Festigerpolymere, üblicherweise in einer Menge von 0,1 bis 10 Gew.-%, bezogen auf das Gesamtgewicht, enthalten.

Die erfindungsgemäßen Polyurethane und Mittel besitzen den Vorteil, daß sie einerseits den Haaren die gewünschte Festigkeit verleihen und andererseits die Polymere leichter auswaschbar (redispergierbar) sind als die Polymere des Standes der Technik. Darüber hinaus lassen sich Haarbehandlungsmittel mit einen VOC-Gehalt von weniger als 60 Gew.-% herstellen, selbst wenn sie als Haarspray formuliert sind.

Die nachfolgenden Beispiele erläutern die Erfindung.

### Beispiel 1

### Polyurethanherstellung:

In einem 4-Halskolben, der mit Rührer, Tropftrichter, Thermometer, Rückflußkühler und einer Vorrichtung für das Arbeiten unter Stickstoff ausgestattet war, wurden 0,5 Mol Polyesterdiol (Mn = 1.000 g/Mol), (hergestellt aus Isophthalsäure, Adipinsäure und Hexandiol), 0,05 Mol Polyethylenglykol (Mn 1500) und 1,25 Mol Dimethylolpropansäure in Methylethylketon unter Erhitzen auf eine Temperatur von 80°C gebracht und unter Rühren gelöst. Sobald sich alles gelöst hatte, wurde das Reaktionsgemisch auf ca. 50°C abgekühlt. Anschließend wurden unter Rühren 1,9 Mol Isophorondiisocyanat zugetropft, wobei die Reaktionstemperatur anstieg. Bei einer Innentemperatur von 90°C wurde das Reaktionsgemisch dann so lange gerührt, bis der Isocyanatgruppengehalt des Gemisches praktisch konstant blieb. Danach kühlte man das Reaktionsgemisch auf Umgebungstemperatur ab und tropfte bei dieser Temperatur 116,5 g Casein in Form einer 15 %igen wäßrigen Casein-Triethanolamin-Lösung (12:1 Gew.:Gew.) zu. Das Reaktionsgemisch wurde dann noch so lange bei Umgebungstemperatur gerührt, bis keine Isocyanatgruppen mehr nachweisbar waren. Anschließend gab man Wasser zum Reaktionsgemisch und neutralisierte das Reaktionsprodukt mit 2-Amino-2-methylpropanol. Das Methylethylketon wurde dann im Vakuum bei 40°C abdestilliert, wobei man eine wäßrige Dispersion des Pfropfpolymere (IV, s. Tab.) erhielt, welche für die in dem nachfolgenden Beispiel 2 beschriebenen Versuche verwendet wurde. Ein trockenes Produkt kann durch Sprühtrocknung (unter Vakuum/ Temp. ∼80°C) erhalten werden.

Analog wurden die anderen Polyurethane gemäß der Tabelle hergestellt.

### Beispiel 2

Handpumpenspray-Formulierung mit einem VOC-Gehalt von 55 Gew.-%:

| | |
|---|---|
| Pfropfpolymere gemäß Beispiel 1 (Feststoffgehalt) | 5 Gew.-% |
| Wasser | 40 Gew.-% |
| Ethanol | 55 Gew.-% |
| Parfüm, Tensid | q.s. |

Die so formulierten Haarsprays wurden auf ihre Festigungswirkung untersucht.

Curl-retention = Festigungswirkung der Haarsträhnen in Lockenform bei hoher Luftfeuchte (90 %):

Die Curl-Retention ist ein Maß für die Haarfestigungswirkung. Sie wird im Modellversuch gemessen an Haarlocken, erzeugt durch eine übliche Wasserwelle an ca. 15 cm langen Haaren, die mit
5 gew.-%iger (Spray)lösung eines Harzes gemäß Tabelle und zu 95 % teilneutralisiert aus 10 cm Entfernung 4 sec lang besprüht werden. Nach 5-stündiger Behandlung der aufgehängten Locken in einer Klimakammer (25°C, 90 % relative Luftfeuchte) wird die relative Verformung (Aufweitung) der Locken, bezogen auf die ursprüngliche Form, festgestellt. Ein hoher Wert bedeutet hohe Festigungswirkung, d.h. 100 % wäre Erhalt der ursprünglichen Form der aufgehängten Locke, 0 % wäre ein völlig gestrecktes Haar.

### Biegefestigkeit

Die Messung erfolgt mit 10 verschiedenen Haarsträhnen, die etwa gleich schwer (2 g) und gleich lang (24 cm) sind. Es handelt sich um mitteleuropäisches braunes Haar. Die Haarsträhnen werden 1 Std. in eine Lösung EtOH/H₂O = 1/1 gelegt, danach wird zweimal mit Texapon NSO Lösung (10 % Feststoff) schamponieren und mit 40°C warmem Wasser ausgespült. Die nassen Haarsträhnen werden durchgekämmt und an der Luft bei Raumtemperatur getrocknet. Nach dem Trocknen werden die Haarsträhnen gewogen und in eine 3%ige ethanolische Filmbildnerlösung getaucht, wobei durch mehrmaliges Eintauchen und Herausnehmen eine gleichmäßige Verteilung sichergestellt ist.

Die überschüssige Filmbildnerlösung wird zwischen Daumen und Zeigefinger abgedrückt und anschließend zwischen Filterpapier sorgfältig auf eine Gewichtszunahme von 0,4 bis 0,5 g gedrückt. Danach werden die Haarsträhnen so geformt, daß sie einen runden Querschnitt erhalten. Es erfolgt die Trocknung im Klimaschrank bei 20°C und 75 % relativer Luftfeuchtigkeit. Nach 12 Stunden werden die Strähnen aus dem Klimaschrank genommen und sofort der Härtemessung unterzogen. Die Haarsträhne wird auf 2zylindrische Rollen (Durchmesser 6 mm) gelegt, die waagrecht zueinander im Abstand von 9 cm angebracht sind. Genau in der Mitte zwischen den beiden Auflagen wird nun von oben durch eine zylindrische Rolle (Durchmesser 6 mm) so lange mit stetig steigender Kraft auf die Haarsträhnen gedrückt, bis diese durchbrechen. Die zum Durchbrechen erforderliche Kraft wird durch Auswiegen der benötigten Masse gemessen. Nach dem Durchbrechen der Haarsträhne wird die Strähne entlastet, wodurch sich die Haarsträhne wieder streckt. Nun wird erneut stetig die Kraft erhöht, bis die Strähne zum zweiten Mal durchbricht.

Die Festigungswirkung der einzelnen erfindungsgemäßen Pfropfpolymere (I bis VII) ist in der Tabelle dargestellt.

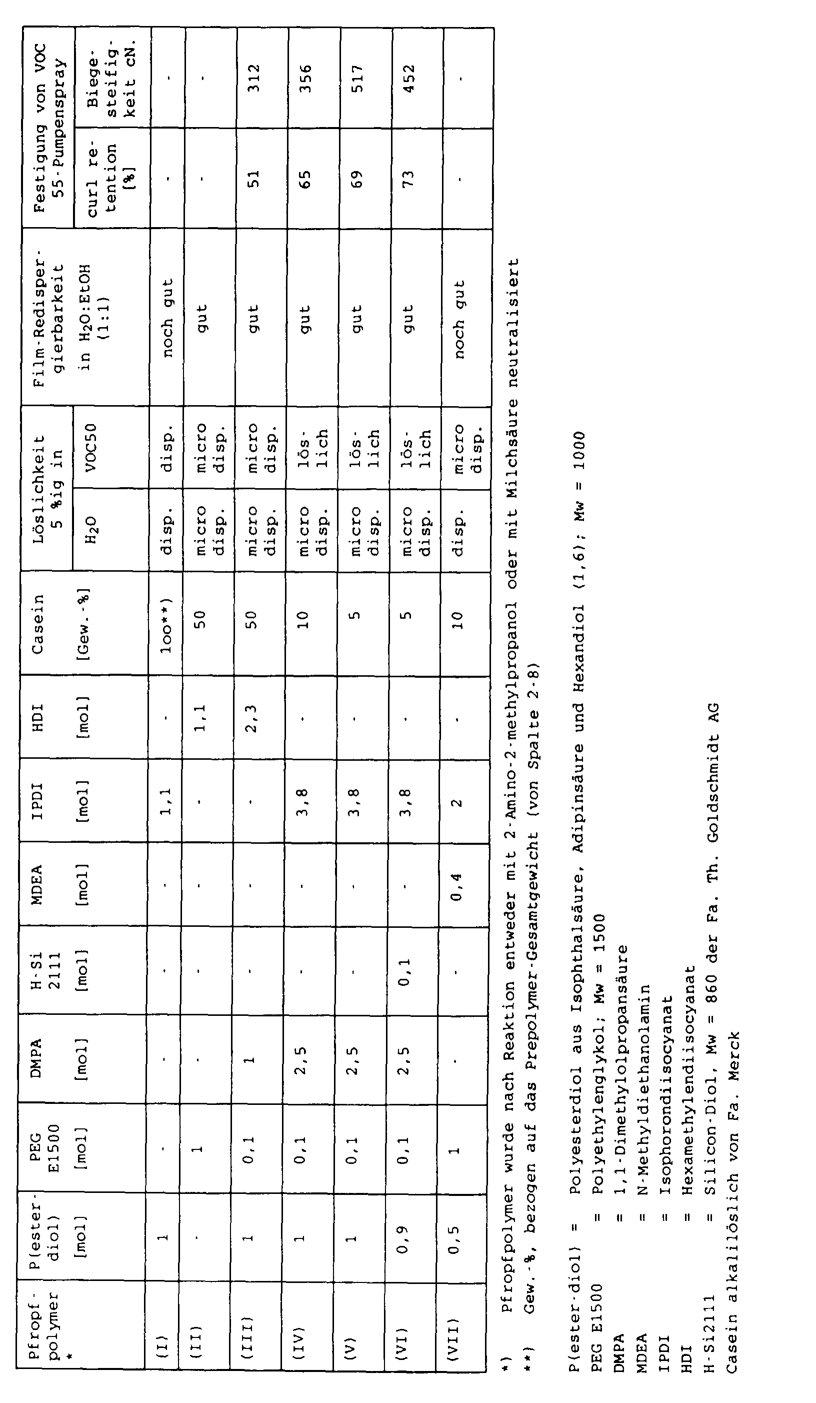

## Patentansprüche

1. Wasserlösliche oder wasserdispergierbare Pfropfpolymere aus
A) einem in Wasser löslichen oder dispergierbaren Polyurethanpräpolymer mit endständigen Isocyanatgruppen und
B) einem freie Aminogruppen enthaltenden Protein,
und die Salze davon.

2. Pfropfpolymere nach Anspruch 1 auf Basis von Polyurethanpräpolymeren aus
a) mindestens einer Verbindung, die zwei oder mehrere aktive Wasserstoffatome pro Molekül enthält,
b) mindestens einer Verbindung, die zwei oder mehrere aktive Wasserstoffatome und mindestens eine Säure-, eine tertiäre Amin- oder eine ionogene bzw. ionische Gruppe pro Molekül aufweist, und
c) mindestens einem Diisocyanat.

3. Pfropfpolymere nach Anspruch 2, wobei das Verhältnis von NCO-Äquivalent zu Äquivalent aktivem Wasserstoff in A) > 1:1 bis 1,2:1 beträgt.

4. Pfropfpolymere nach Anspruch 2 oder 3, wobei es sich bei den ionogenen bzw. ionischen Gruppen der Komponente b) um Carboxylatgruppen und/oder Sulfonatgruppen oder um stickstoffhaltige Gruppen handelt.

5. Pfropfpolymere nach Anspruch 4, wobei es sich bei der Verbindung b) um Dimethylolpropansäure handelt.

6. Pfropfpolymere nach einem der Ansprüche 2 bis 5, wobei die Komponente a) mindestens 30 Gew.-%, vorzugsweise 40 bis 90 Gew.-%, bezogen auf das Gesamtgewicht der Komponenten a) und b), eines Polyesterdiols umfaßt.

7. Pfropfpolymere nach einem der Ansprüche 2 bis 6, wobei die Komponente a) bis zu 50 Gew.-%, bezogen auf das Gesamtgewicht der Komponenten a) und b), einer Silikonverbindung der Formel 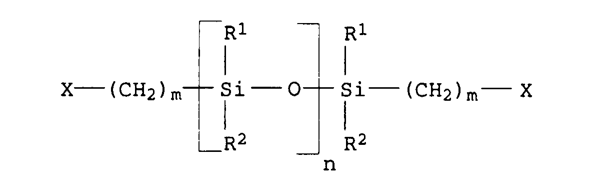 umfaßt,
worin
R¹ und R², die gleich oder verschieden sein können, für C₁-C₄-Alkyl, Benzyl oder Phenyl stehen,
die Reste X die gleich oder verschieden sein können, für OH oder NH₂ stehen,
m für 2 bis 10 steht und
n für 3 bis 50 steht.

8. Pfropfpolymere nach einem der vorhergehenden Ansprüche, wobei als Protein B) ein Casein oder ein Hydrolyseprodukt eines Caseins eingesetzt wird.

9. Verfahren zur Herstellung der Pfropfpolymere nach einem der Ansprüche 1 bis 7, dadurch gekennzeichnet, daß man das Polyurethanpräpolymer mit einem freie Aminogruppen enthaltenden Protein zur Reaktion bringt.

10. Verfahren nach Anspruch 9, dadurch gekennzeichnet, daß man das Protein in Form einer wäßrigen oder wäßrig-alkoholischen Lösung einsetzt.

11. Verfahren nach Anspruch 10, dadurch gekennzeichnet, daß die Proteinlösung ein tertiäres Amin enthält.

12. Verwendung der Pfropfpolymere nach einem der Ansprüche 1 bis 7 als Hilfsmittel in der Kosmetik, insbesondere in der Haarkosmetik.

13. Verwendung nach Anspruch 12, wobei die Pfropfpolymere als Haarfestiger zur Anwendung kommen.

14. Haarbehandlungsmittel, insbesondere in Form eines Haarsprays, das wenigstens ein Pfropfpolymer nach einem der Ansprüche 1 bis 7 enthält.

15. Haarbehandlungsmittel nach Anspruch 14, enthaltend
a) 0,2 bis 20 Gew.-%, bezogen auf das Gesamtgewicht, eines Pfropfpolymers nach Anspruch 1 bis 7 und
b) 0,1 bis 10 Gew.-% eines handelsüblichen Haarfestigerpolymers.

16. Verwendung der Pfropfpolymere gemäß Anspruch 1 bis 7 als redispergierbare Beschichtungsmittel für Pharma-, Papier-, Textil- oder Lederverarbeitung.

## Claims

1. A water-soluble or water-dispersible graft polymer of
A) a water-soluble or -dispersible polyurethane prepolymer having terminal isocyanate groups and
B) a protein containing free amino groups,
or a salt thereof.

2. A graft polymer as claimed in claim 1, based on polyurethane prepolymers of
a) at least one compound containing two or more active hydrogen atoms per molecule,
b) at least one compound containing two or more active hydrogen atoms and at least one acid group, tertiary amine group or ionogenic or ionic group per molecule, and
c) at least one diisocyanate.

3. A graft polymer as claimed in claim 2, wherein the ratio of NCO equivalent to equivalent of active hydrogen in A) is from > 1:1 to 1.2:1.

4. A graft polymer as claimed in claim 2 or 3, wherein the ionogenic and/or ionic groups of component b) are carboxylate groups and/or sulfonate groups or nitrogen-containing groups.

5. A graft polymer as claimed in claim 4, wherein the compound b) is dimethylolpropanoic acid.

6. A graft polymer as claimed in any one of claims 2 to 5, wherein component a) comprises at least 30 % by weight, preferably from 40 to 90 % by weight, based on the overall weight of components a) and b), of a polyesterdiol.

7. A graft polymer as claimed in any one of claims 2 to 6, wherein component a) comprises up to 50 % by weight, based on the overall weight of components a) and b), of a silicone compound of the formula 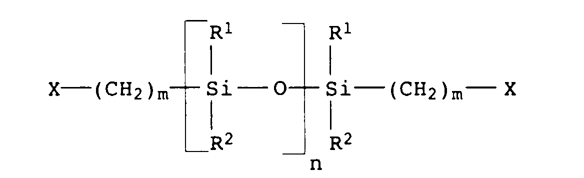 in which
R¹ and R² are identical or different and are C₁-C₄-alkyl, benzyl or phenyl,
the radicals X, which can be identical or different, are OH or NH₂,
m is from 2 to 10, and
n is from 3 to 50.

8. A graft polymer as claimed in any one of the preceding claims, wherein a casein or a hydrolysis product of a casein is employed as protein B).

9. A process for preparing a graft polymer as claimed in any one of claims 1 to 7, which comprises reacting the polyurethane prepolymer with a protein which contains free amino groups.

10. A process as claimed in claim 9, wherein the protein is employed in the form of an aqueous or aqueous-alcoholic solution.

11. A process as claimed in claim 10, wherein the protein solution comprises a tertiary amine.

12. The use of a graft polymer as claimed in any one of claims 1 to 7 as an auxiliary in cosmetology, especially in hair cosmetology.

13. The use as claimed in claim 12, where the graft polymers are employed as hairsetting agents.

14. A hair treatment composition, especially in the form of a hair spray, which comprises at least one graft polymer as claimed in any one of claims 1 to 7.

15. A hair treatment composition as claimed in claim 14, comprising
a) from 0.2 to 20 % by weight, based on the overall weight, of a graft polymer as claimed in claim 1 to 7 and
b) from 0.1 to 10 % by weight of a commercially available hairsetting polymer.

16. The use of a graft polymer as claimed in claim 1 to 7 as a redispersible coating agent for the processing of pharmaceuticals, paper, textiles or leather.

## Revendications

1. Polymères greffés solubles dans l'eau ou dispersables dans l'eau à base de
A) un polymère de polyuréthanne soluble ou dispersable dans l'eau, ayant des groupes isocyanate terminaux et
B) une protéine contenant des groupes amino libres, et leurs sels.

2. Polymères greffés selon la revendication 1, à base de prépolymères de polyuréthanne de
a) au moins un composé qui contient deux ou plus de deux atomes d'hydrogène actif par molécule,
b) au moins un composé qui présente deux ou plus de deux atomes d'hydrogène actif et au moins un groupe acide, un groupe amine tertiaire ou un groupe ionogène ou ionique par molécule, et
c) au moins un diisocyanate.

3. Polymères greffés selon la revendication 2, dans lesquels le rapport d'équivalent de NCO à l'équivalent d'hydrogène actif dans A) est supérieur à 1:1 à 1,2:1.

4. Polymères greffés selon la revendication 2 ou 3, dans lesquels il s'agit pour les groupes ionogènes ou ioniques du composant b) de groupes carboxylate et/ou de groupes sulfonate ou de groupes contenant de l'azote.

5. Polymères greffés selon la revendication 4, dans lesquels il s'agit pour le composé b) d'acide diméthylolpropanoïque.

6. Polymères greffés selon l'une des revendications 2 à 5, dans lesquels le composant a) comprend au moins 30% en poids, de préférence 40 à 90% en poids, par rapport à la masse totale des composants a) et b), d'un polyesterdiol.

7. Polymères greffés selon l'une des revendications 2 à 6, dans lesquels le composant a) comporte jusqu'à 50% en poids, par rapport à la masse totale des composants a) et b), d'un composé de silicone de formule 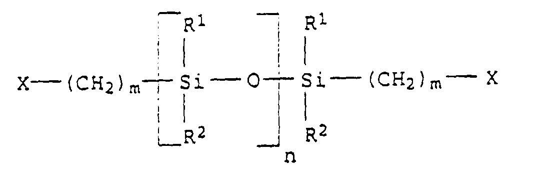 où
R¹ et R², qui peuvent être identiques ou différents, désignent un radical alkyle en C₁-C₄, benzyle ou phényle,
les restes X, qui peuvent être identiques ou différents, représentent OH ou NH₂,
m vaut de 2 à 10 et
n vaut de 3 à 50.

8. Polymères greffés selon l'une des revendications précédentes, dans lesquels se trouve comme protéine B) une caséine ou un produit d'hydrolyse d'une caséine.

9. Procédé pour la préparation des polymères greffés selon l'une des revendications 1 à 7, caractérisé par le fait qu'on fait réagir le prépolymère de polyuréthanne avec une protéine contenant des groupes amino libres.

10. Procédé selon la revendication 9, caractérisé par le fait qu'on introduit la protéine sous forme d'une solution aqueuse ou aqueuse-alcoolique.

11. Procédé selon la revendication 10, caractérisé par le fait que la solution de protéine contient une amine tertiaire.

12. Utilisation des polymères greffés selon l'une des revendications 1 à 7 comme adjuvant en cosmétique, en particulier en cosmétique capillaire.

13. Utilisation selon la revendication 12, dans laquelle les polymères greffés sont employés comme agents de renforcement du cheveu.

14. Agents pour le traitement du cheveu, en particulier sous la forme d'un spray capillaire, qui contient au moins un polymère greffé selon l'une des revendications 1 à 7.

15. Agents pour le traitement du cheveu selon la revendication 14, contenant
a) 0,2 à 20% en poids, par rapport à la masse totale, d'un polymère greffé selon la revendication 1 à 7, et
b) 0,1 à 10% en poids d'un polymère commercial renforçateur capillaire.

16. Utilisation des polymères greffés selon la revendication 1 à 7 comme agents d'enduction redispersables pour traitement des produits pharmaceutiques, du papier, des textiles ou du cuir.
